# EUROPEAN PATENT SPECIFICATION

(11) **EP 4 014 048 B1**
(45) Date of publication and mention of the grant of the patent: **08.01.2025**
(21) Application number: 20751577.6
(22) Date of filing: 12.08.2020
(51) Int. Cl.: G01N 33/68

(54) **NOVEL DIAGNOSTIC MARKER FOR CREUTZFELDT-JAKOB DISEASE AND ALZHEIMER'S DISEASE**
NEUARTIGER DIAGNOSTISCHER MARKER FÜR DIE CREUTZFELDT-JAKOB-KRANKHEIT UND MORBUS ALZHEIMER
NOUVEAU MARQUEUR DE DIAGNOSTIC DE LA MALADIE DE CREUTZFELDT-JAKOB ET DE LA MALADIE D'ALZHEIMER

(30) Priority: 12.08.2019 EP 19191213
(43) Date of publication of application: 22.06.2022
(73) Proprietor: Otto, Markus, 89077 Ulm (DE); Öckl, Patrick Christian, 88447 Warthausen (DE); Halbgebauer, Steffen, 89231 Neu-Ulm (DE)
(72) Inventor: Otto, Markus, 89077 Ulm (DE); Öckl, Patrick Christian, 88447 Warthausen (DE); Halbgebauer, Steffen, 89231 Neu-Ulm (DE)
(74) Representative: Klöckner, Christoph
(86) International application number: PCT/EP2020/072559
(87) International publication number: WO 2021/028452

(56) References cited:
- WO-A1-2018/005791
- WO-A1-2019/099732
- US-A1- 2005 176 078
- GELON PAULINE A. ET AL: "Synaptic dysfunction in ALS and FTD: anatomical and molecular changes provide insights into mechanisms of disease", FRONTIERS IN MOLECULAR NEUROSCIENCE, vol. 15, 3 October 2022 (2022-10-03), XP093102428, Retrieved from the Internet <URL:https://www.ncbi.nlm.nih.gov/pmc/articles/PMC9575515/pdf/fnmol-15-1000183.pdf> DOI: 10.3389/fnmol.2022.1000183
- OECKL PATRICK ET AL: "Targeted Mass Spectrometry Suggests Beta-Synuclein as Synaptic Blood Marker in Alzheimer's Disease", JOURNAL OF PROTEOME RESEARCH, vol. 19, no. 3, 26 February 2020 (2020-02-26), pages 1310 - 1318, XP093102540, ISSN: 1535-3893, DOI: 10.1021/acs.jproteome.9b00824
- LORENZO BARBA: "Serum [beta]-synuclein, neurofilament light chain and glial fibrillary acidic protein as prognostic biomarkers in moderate-to-severe acute ischemic stroke", SCIENTIFIC REPORTS, vol. 13, no. 1, 28 November 2023 (2023-11-28), US, pages 20941, XP093159461, ISSN: 2045-2322, Retrieved from the Internet <URL:https://www.ncbi.nlm.nih.gov/pmc/articles/PMC10684607/pdf/41598_2023_Article_47765.pdf> DOI: 10.1038/s41598-023-47765-7
- REBECCA HALBGEBAUER: "Neurochemical Monitoring of Traumatic Brain Injury by the Combined Analysis of Plasma Beta-Synuclein, NfL, and GFAP in Polytraumatized Patients", INTERNATIONAL JOURNAL OF MOLECULAR SCIENCES, vol. 23, no. 17, 25 August 2022 (2022-08-25), Basel, CH, pages 9639, XP093159463, ISSN: 1422-0067, Retrieved from the Internet <URL:https://www.ncbi.nlm.nih.gov/pmc/articles/PMC9456193/pdf/ijms-23-09639.pdf> DOI: 10.3390/ijms23179639
- SAMIR ABU-RUMEILEH: "Plasma beta-synuclein, GFAP, and neurofilaments in patients with malignant gliomas undergoing surgical and adjuvant therapy", ANNALS OF CLINICAL AND TRANSLATIONAL NEUROLOGY, vol. 10, no. 10, 23 August 2023 (2023-08-23), GB, pages 1924 - 1930, XP093159458, ISSN: 2328-9503, Retrieved from the Internet <URL:https://onlinelibrary.wiley.com/doi/pdfdirect/10.1002/acn3.51878> DOI: 10.1002/acn3.51878
- PATRICK OECKL ET AL: "Alpha-, Beta-, and Gamma-synuclein Quantification in Cerebrospinal Fluid by Multiple Reaction Monitoring Reveals Increased Concentrations in Alzheimer's and Creutzfeldt-Jakob Disease but No Alteration in Synucleinopathies", MOLECULAR & CELLULAR PROTEOMICS, vol. 15, no. 10, 1 October 2016 (2016-10-01), US, pages 3126 - 3138, XP055659357, ISSN: 1535-9476, DOI: 10.1074/mcp.M116.059915
- K. BEYER ET AL: "The decrease of beta-synuclein in cortical brain areas defines a molecular subgroup of dementia with Lewy bodies", BRAIN, vol. 133, no. 12, 19 October 2010 (2010-10-19), pages 3724 - 3733, XP055222665, ISSN: 0006-8950, DOI: 10.1093/brain/awq275

## Description

### Technical field

The present invention relates to a method of diagnosis or assessing the status of a disease or diseases associated with synaptic degeneration, as further defined in the claims, in particular of Creutzfeldt-Jakob-Disease or Alzheimer's Disease, and to the use of β-synuclein as a biomarker for diagnosing or assessing the status of the said diseases associated with synaptic degeneration as further defined in the claims, in particular of Creutzfeldt-Jakob-Disease or Alzheimer's Disease.

### Background of the Invention

Synapses, the functional connections between neurons and other neurons or cells, are essential sites for memory formation. Their dysfunction or degeneration is associated with memory impairment, a cardinal clinical feature of Alzheimer's disease (AD). AD is an example for a neurodegenerative disease characterized by a progressive loss of cognitive function due to the degeneration of related brain regions such as the hippocampus with yet unknown etiology. Synaptic loss is an early event in AD pathogenesis and already starts in the prodromal phase of the disease. Synaptic degeneration is a major hallmark of Alzheimer's disease (AD) and the best pathological correlate of cognitive dysfunction. It shows stronger correlation with cognitive dysfunction than other key neuropathological features of AD including plaques, tangles and neuronal loss.

Fluid biomarkers are a desired tool to evaluate and monitor synaptic degeneration for diagnosis, in clinical trials and patient follow-up and they are more cost effective than PET imaging.

Several synaptic marker proteins have previously been described in cerebrospinal fluid (CSF) such as neurogranin, SNAP-25 and GAP-43 and their CSF levels are altered in AD, however studies of such biomarkers in blood were not successful so far. Neurogranin, as the best characterized synaptic biomarker, is increased in CSF of AD patients but no difference could be observed in blood samples which is attributed to the additional extracerebral synthesis of neurogranin.

Reliable biomarkers combined with clinical examinations not only improve the diagnostic accuracy of neurodegenerative diseases but are also the most promising key in helping clinicians making an accurate predictive diagnosis. Thus, the analysis of surrogate biomarkers in blood or cerebrospinal fluid (CSF), reflecting specific biochemical or structural alterations in the central nervous system (CNS), is most auspicious. For Alzheimer's disease (AD), the measurement of Tau protein and the Amyloid-β peptide 1-42 (Aβ42) in the CSF, is already successfully implemented in the clinic.

However, neither Tau as a general neurodegeneration marker nor Aβ42 as a marker for amyloid deposition reflect the degeneration of synapses occurring in AD or other neurodegenerative diseases. Assessing the synaptic dysfunction, which plays a major role in AD pathogenesis, could be of great benefit not only for the diagnosis but also in monitoring synaptic features of novel drug candidates in clinical trials. Moreover, synaptic loss is often preceding neuronal degeneration thereby already occurring in early AD and even patients with mild cognitive impairment (MCI).

Additionally, the loss of synapses is more closely associated with cognitive deterioration than tangle and plaque pathology in AD. Monitoring this loss by the analysis of a synaptic protein would be of great interest in the struggle for an early diagnosis and/or prognostic statement.

Increased levels of the presynaptic protein β-synuclein (βSyn) in CSF have previously been described in a small cohort of AD patients (cf. Oeckl, P. et al. (2016) Mol. Cell. Proteomics. 15, 3126-3138). Besides, WO 2018/005791 e.g. discloses a method of providing clinical information about TBI and US 2005/176078 e.g. discloses a method of diagnosing a synucleinopathy, both of which methods e.g. involve measuring beta-synuclein. Moreover, it has been described that the decrease of beta-synuclein in cortical brain areas defines a molecular subgroup of dementia with Lewy bodies (cf. Beyer et al. (2010) Brain, 133(12), 3724-33).

βSyn is was shown to be expressed in the central nervous system (CNS) and highly enriched in the hippocampus but its function is not well understood. There is evidence for an involvement in membrane-associated processes in the synapse and an anti-aggregation potential and neuroprotective function has been described. βSyn is present in hippocampal pathology in dementia with Lewy bodies (DLB) and Parkinson's disease. Two mutations in the βSyn gene (V70M, P123H) are associated with DLB suggesting a link to cognitive dysfunction.

The identification of a suitable synaptic biomarker specifically reflecting synaptic degeneration together with reliable methods for detection in body fluids, such as blood, which are more easily accessible than CSF would bear several advantages over detection of biomarkers in CSF including lower costs, less risks and higher convenience for patients and clinicians.

Thus, it is an object of the present invention to provide useful and advantageous methods for diagnosis of neurodegenerative diseases which provide a more exact determination of the state of a disease in an individual patient and at the same time allowing said methods to be carried out with higher convenience, lower prices and reduced risks by making patient sample collection possible at standard points-of-care.

### Summary of the Invention

These objects have been solved by the aspects of the present invention as specified hereinafter.

According to the first aspect of the present invention, a method as defined in the claims for diagnosis of a disease or diseases associated with synaptic degeneration is provided, the method comprising determining the concentration of β-synuclein in a sample of a patient. Likewise, corresponding methods for assessing the status of a disease or diseases associated with synaptic degeneration are provided as defined in the claims.

According to the first aspect of the present invention, the sample of the patient is blood, serum, or plasma, preferably blood, more preferably blood serum, even more preferably the sample of the patient is a blood sample, even more preferably a blood serum sample.

According to the first aspect of the present invention, the method is carried out
*ex vivo.*

According to one preferred embodiment of the first aspect of the present invention, the disease or diseases associated with synaptic degeneration are one or more of Alzheimer's disease, Creutzfeldt-Jakob disease, traumatic brain injury, stroke, or glioma, preferably the disease or diseases associated with synaptic degeneration are Alzheimer's disease and/or Creutzfeldt-Jakob disease, more preferably the disease associated with synaptic degeneration is Alzheimer's disease.

According to a preferred embodiment of the first aspect of the present invention, the method is for assessing the status of the disease or diseases associated with synaptic degeneration, or alternatively a method of classifying a stage, preferably a prognostic stage, of a patient diagnosed with or suspected of having the disease or diseases associated with synaptic degeneration, or alternatively a method of monitoring disease progression in patients diagnosed with or suspected of having the disease or diseases associated with synaptic degeneration.

According to a preferred embodiment of the first aspect of the present invention, the determination of the concentration of β-synuclein involves quantitative mass spectrometry, preferably multiple/selected reaction monitoring (MRM/SRM) or parallel reaction monitoring (PRM), more preferably the determination of the concentration of β-synuclein is carried out by means of multiple/selected reaction monitoring or parallel reaction monitoring, even more preferably by measuring the peptides of aa 46 to 58 (EGVVQGVASVAEK) and/or 61 to 85 (EQASHLGGAVFSGAGNIAAATGLVK) of β-synuclein.

According to a preferred embodiment of the first aspect of the present invention, the cut-off value for diagnosing Alzheimer's disease is at 10 pg/ml of β-synuclein in serum, preferably at 10.6 pg/ml.

According to another preferred embodiment of the first aspect of the present invention, the cut-off value for diagnosing Creutzfeldt-Jakob disease is at 39,8 pg/ml, preferably as determined by Ser MRM (Youden Index).

According to one preferred embodiment of the first aspect of the present invention, the determination of the concentration of β-synuclein involves single-molecule arrays (Simoa), preferably the determination of the concentration of β-synuclein is carried out by means of single-molecule arrays, more preferably a step of immunoprecipitation of β-synuclein is carried out before determination of β-synuclein concentration via single-molecule arrays (Simoa).

According to a more preferred embodiment of the preceding preferred embodiment of the first aspect of the present invention, the setup of the single-molecule arrays employs a monoclonal anti β-synuclein antibody coupled to carboxylated paramagnetic beads and/or a biotinylated monoclonal anti α- and β-synuclein antibody as a detection antibody and/or streptavidin-β-galactosidase (SBG) as enzyme reagent and/or Resorufin β-D Galactopyranoside as enzyme substrate.

According to a preferred embodiment of the first aspect of the present invention, the method is used for discriminating Creutzfeldt-Jakob disease, Alzheimer's disease, behavioral variant frontotemporal dementia, and amyotrophic lateral sclerosis from each other, wherein the method combines determination of neurofilament (NfL) and β-synuclein levels in blood, preferably wherein neurofilament (NfL) levels in blood are increased over a reference value for healthy subjects.

According to the second aspect of the present invention, the use of β-synuclein as a biomarker is provided for diagnosing or assessing the status of a disease or diseases associated with synaptic degeneration, preferably for evaluating response to therapy of a patient diagnosed with or suspected of having a disease or diseases associated with synaptic degeneration, and/or for classifying a stage of a patient diagnosed with or suspected of having a disease or diseases associated with synaptic degeneration, and/or for monitoring disease control of a patient diagnosed with or suspected of having a disease or diseases associated with synaptic degeneration, and/or for monitoring disease progression of a patient diagnosed with or suspected of having a disease or diseases associated with synaptic degeneration, as further defined in the claims.

According to a preferred embodiment of the second aspect of the present invention, β-synuclein is used as biomarker in serum of a patient.

### Description of Figures

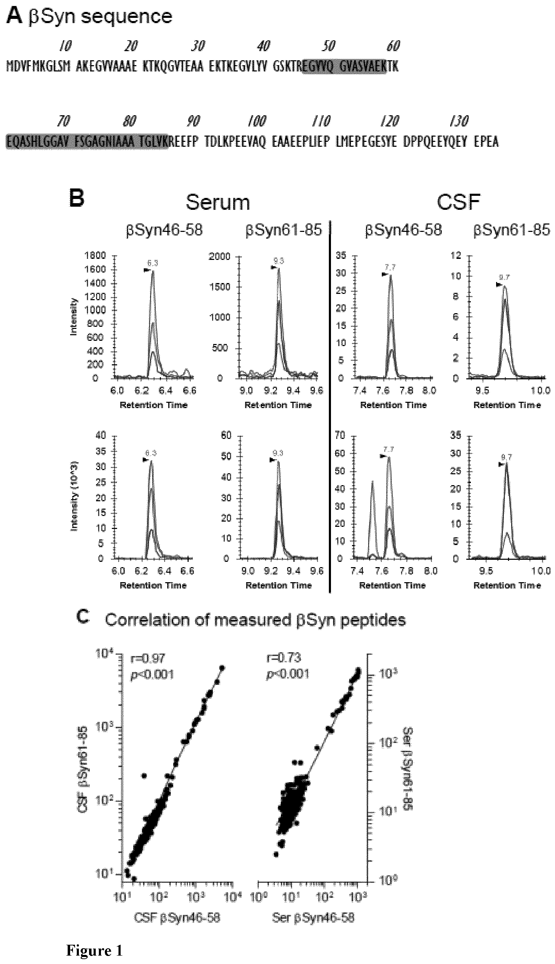

Figure 1 shows (A) the amino acid sequence of βSyn (UniProt Q16143) and the proteotypic tryptic peptides of βSyn measured by MRM (βSyn46-58 and βSyn61-85) highlighted in green, (B) representative chromatograms of βSyn peptides in CSF and serum measured by MRM (Light (endogenous) peptide (Top), heavy peptide (Bottom)), wherein colors are the different MRM transitions measured, and (C) scatter plot of the concentration (pg/mL) of the two measured βSyn peptides in CSF and serum (Ser).

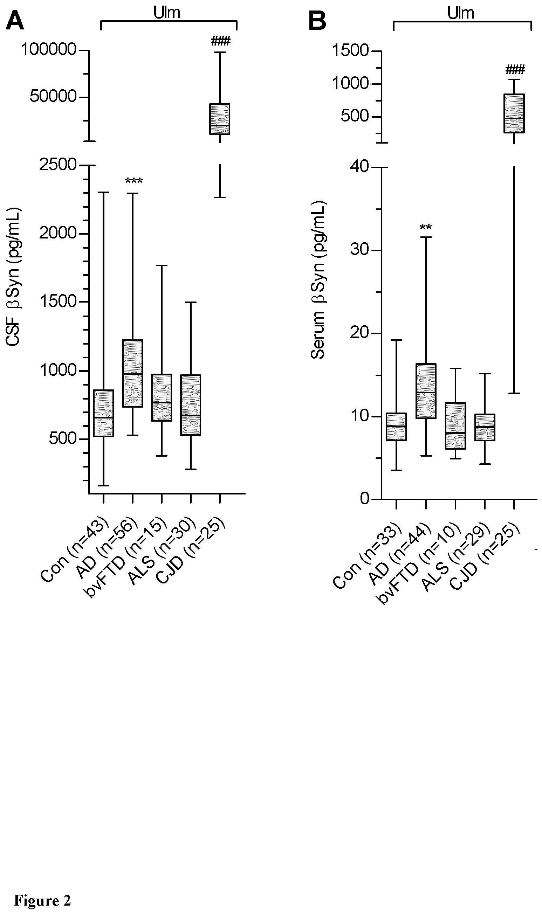

Figure 2 shows the measurement of βSyn in (A) CSF and (B) serum by MRM in a discovery cohort of patients (Ulm). Patients are controls (Con), patients with Alzheimer's disease (AD), behavioral variant of frontotemporal dementia (bvFTD), amyotrophic lateral sclerosis (ALS) and Creutzfeldt-Jakob disease (CJD). Boxes are medians and interquartile ranges, whiskers are min and max. **p<0.01 vs. Con, ***p<0.001 vs. Con, ^{###}p<0.001 vs. all other groups.

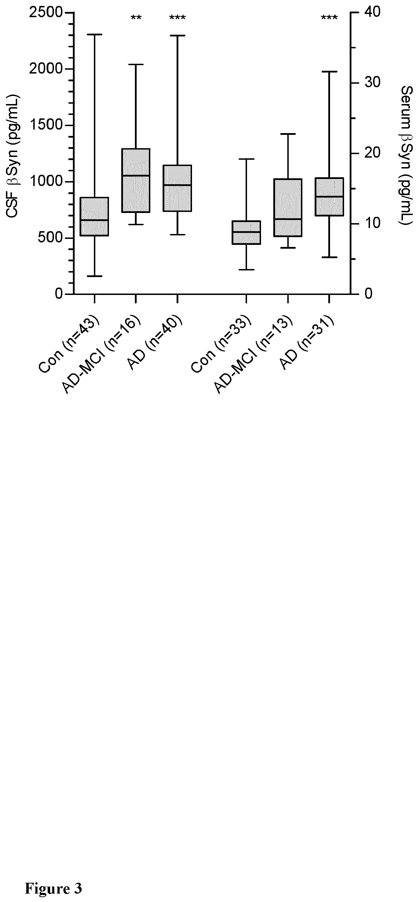

Figure 3 shows the CSF and serum βSyn concentrations measured by MRM in the discovery cohort (Ulm). Alzheimer's disease patients were divided into patients with mild cognitive impairment (AD-MCI, CDR<2.5), representing the early disease phase, and patients with manifest dementia (AD, CDR≥2.5). Con: non-neurodegenerative control patients. Boxes are medians and interquartile ranges, whiskers are min and max. **p<0.01 vs. Con, ***p<0.001 vs. Con.

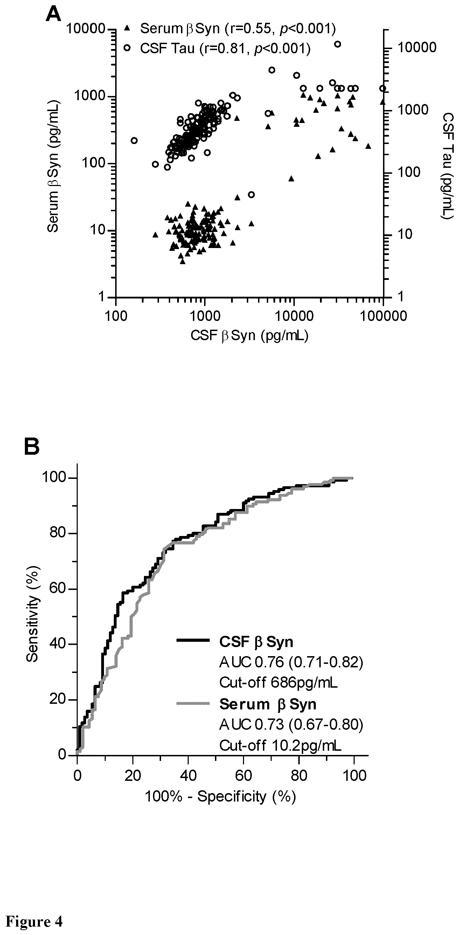

Figure 4 shows (A) scatter plot and correlation analysis (Spearman) of CSF βSyn concentration with serum βSyn (n=140) and CSF Tau (n=147) concentration in the discovery cohort (Ulm), and (B) receiver operating characteristic (ROC) curve analysis of CSF (n=255, black line) and serum βSyn (n=221, gray line) for the discrimination of Alzheimer's disease patients and controls including patients from all three cohorts. The AUC (area under the ROC curve) is given with the 95%CI in brackets. Cut-offs were calculated using the Youden index.

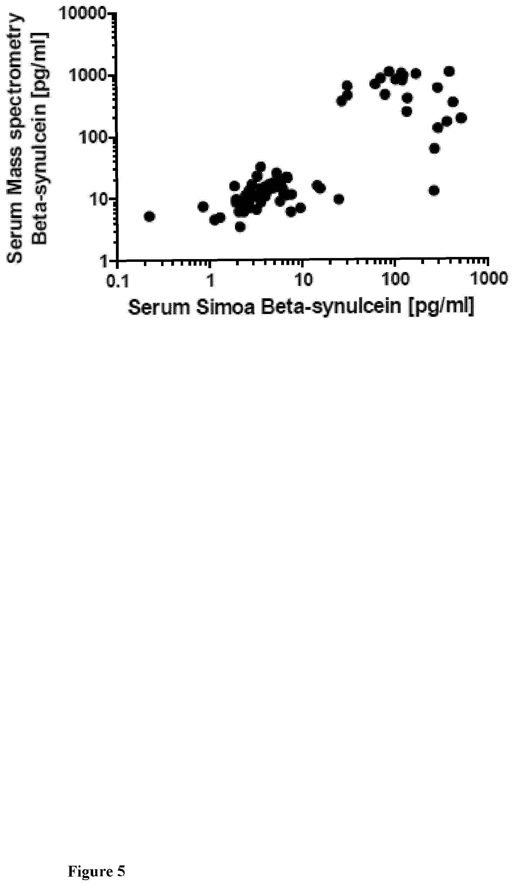

Figure 5 shows the association between serum beta-synuclein Simoa and mass spectrometry (r = 0.76 (Cl: 0.65- 0.84), p<0.0001).

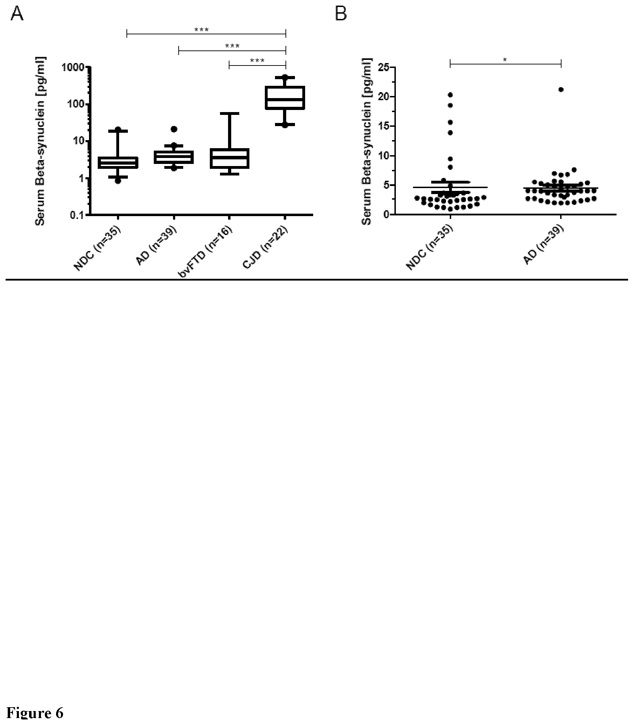

Figure 6 shows (A) the analysis of serum beta-synuclein levels of in total 112 samples. Results are shown as box plots with median concentration, 25% and 75% percentile, and 5% and 95% whiskers on a logarithmic scale, and (B) the comparison of serum beta-synuclein levels between NDC and AD subjects. Findings are shown as scatter dot plots with mean ± SEM. Asterisks indicate significant differences between groups (*p<0.05, ***p<0.0001). AD, Alzheimer's disease; bvFTD, behavioral variant frontotemporal dementia; CJD, Creutzfeldt-Jakob disease; NDC, non-demented control; CSF, cerebrospinal fluid; SEM, standard error of the mean.

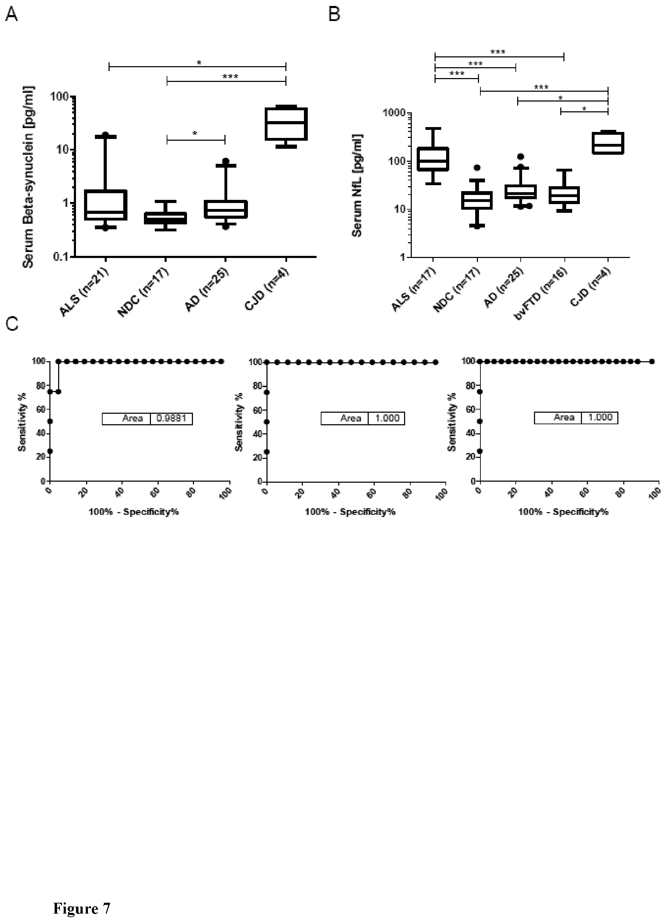

Figure 7 shows (A) serum beta-synuclein levels of Simoa measurement with previous IP, and (B) corresponding Serum NfL levels, wherein results are shown as box plots with median concentration, 25% and 75% percentile, and 5% and 95% whiskers on a logarithmic scale. Asterisks indicate significant differences between groups (*p<0.05, ***p<0.0001). (C) ROC analyses for the discrimination of CJD and ALS (left)/NDC (middle)/AD (right). AD, Alzheimer's disease; bvFTD, behavioral variant frontotemporal dementia; CJD, Creutzfeldt-Jakob disease; CSF, cerebrospinal fluid; IP, immunoprecipitation; NDC, non-demented control; NfL, neurofilament light chain.

### Detailed Description of the Invention

The present inventors have dedicated themselves to solving the problem of the present invention and were successful to find novel methods for a reliable diagnosis of neurodegenerative diseases providing advantageous determination of the state of a disease with increased convenience, lower prices and reduced risks and which make patient sample collection possible at standard points-of-care.

Using quantitative mass spectrometry (MRM), the inventors demonstrate that increased concentrations of the presynaptic protein β-synuclein can be detected in blood samples of AD and CJD patients. These findings suggest β-synuclein as a blood marker for synaptic degeneration which may be used in differential diagnosis of patients suffering from or suspected to suffer from a neurodegenerative disease, clinical trials involving such patients, patient follow-up and other indications.

Herein, a method for the quantification of the presynaptic protein βSyn in human serum in the very low pg/mL range by mass spectrometry is provided and successfully correlated to indications and symptoms of neurogenerative diseases such as AD and CJD. This is of high clinical relevance since no synaptic marker in blood is available so far.

The postsynaptic protein neurogranin can be measured in blood as well but it is unchanged in AD patients and therefore not a suitable synaptic marker for neurodegenerative diseases in blood. This is attributed to the extracerebral synthesis of neurogranin masking alterations of brain-derived neurogranin in blood. Other synaptic proteins have not been measured in blood so far.

In addition, synaptic dysfunction appears to be a valuable read-out for clinical drug trials due to its importance in AD pathogenesis and correlation with clinical symptoms. Blood collection is faster, more cost effective and more convenient for patients than CSF collection which is why serum βSyn measurement is advantageous to CSF βSyn or other synaptic CSF markers. It enables a more close-meshed monitoring of synaptic degeneration in longitudinal studies and the screening of larger populations for patient selection in clinical trials.

Also in patients suffering from CJD patients,a massive increase of βSyn in CSF and serum could be observed and may be used to discriminate CJD from AD and the other diseases with nearly 100% sensitivity and specificity. This observation is in agreement with synaptic degeneration as a primary neuropathological feature of CJD and the synaptic location of the prion protein whose misfolding drives the degenerative process in CJD. Thus, βSyn is an ideal diagnostic biomarker for CJD in blood.

Another candidate would be the neurofilament light chain (NfL) which is also increased in ALS. βSyn is not changed in ALS and therefore, it is a more suitable blood marker for CJD diagnosis than NfL. The unchanged CSF and serum βSyn levels in ALS also indicate that βSyn is not just a general marker of neurodegeneration. Combining NfL and βSyn measurements in blood could lead to an even higher potential for diagnosis of neurodegenerative diseases. Since synaptic dysfunction and degeneration is associated with many other neurological diseases or insults of the CNS such as traumatic brain injury, stroke or alcoholism, a synaptic marker in blood will be useful for diagnosis, prognosis, personalized treatment or drug development in more neurodegenerative diseases.

Thus, the present invention provides a method for diagnosis of a disease or diseases associated with synaptic degeneration, the method comprising determining the concentration of β-synuclein in a sample of a patient as defined in the claims. In a preferable embodiment of the present invention, the method for diagnosis is for diagnosis in a mammal, more preferably in a human patient.

The full length amino acid sequence of β-synuclein in humans can be obtained under the UniProt accession number Q16143. Herein, the amino acid sequence of β-synuclein may be referred to as SEQ ID No. 1.

The patient sample to be used in the present invention preferably has not been obtained by lumbar puncture. More preferably, the sample of the patient does not contain molecular markers which are found exclusively in CSF but not in other body fluids.

Based on the specific advantage of the method of the present invention, the sample of the patient comprises one or more of blood, serum, or plasma. In fact, any body fluid in which β-synuclein can be detected could in principle be used as the sample of the patient. More preferred as being comprised in the patient sample are blood and even more preferred is blood serum of a patient. In an even more preferred embodiment, the sample of a patient to be analysed within the method of the present invention is a blood sample, particularly preferably a blood serum sample.

The method of the present invention is carried out ex *vivo.* In the context of the present invention, the method is used in cases of diseases associated with synaptic degeneration which are one or more of Alzheimer's disease, Creutzfeldt-Jakob disease, traumatic brain injury, stroke, or glioma. However, it is pointed out that due to the specific property of the method to directly reflect synaptic degeneration, the method may be useful for any type of disease or condition which involves synaptic degeneration.

Still, more preferably in context of the present invention, the disease or diseases associated with synaptic degeneration are Alzheimer's disease and/or Creutzfeldt-Jakob disease. Particularly preferably, the disease associated with synaptic degeneration is Alzheimer's disease

The method of the present invention is useful for all types and steps of determination of disease parameters and indications which may contribute to or be considered as a "diagnosis". Preferably, the method serves for assessing the status of the disease or diseases associated with synaptic degeneration.

Also preferably, the method serves for determining response to therapy of a patient diagnosed with or suspected of having the disease or diseases associated with synaptic degeneration. Also preferably, the method is for classifying a stage, preferably a prognostic stage, of a patient diagnosed with or suspected of having the disease or diseases associated with synaptic degeneration. Alternatively preferably, the method is for monitoring disease progression in patients diagnosed with or suspected of having the disease or diseases associated with synaptic degeneration.

Preferably herein, the determination of the concentration of β-synuclein involves quantitative mass spectrometry, more preferably multiple/selected reaction monitoring (MRM/SRM), and/or parallel reaction monitoring (PRM). Even more preferably, the determination of the concentration of β-synuclein is carried out by means of multiple/selected reaction monitoring or parallel reaction monitoring, particularly preferably by measuring the peptides of aa 46 to 58 (SEQ ID No. 2; EGVVQGVASVAEK) and/or 61 to 85 (SEQ ID No. 3;
EQASHLGGAVFSGAGNIAAATGLVK) of β-synuclein by means of multiple reaction monitoring.

The diagnostic cut-off values were calculated from serum MRM values using the Youden index, wherein a p-value <0.05 was regarded as significant. A preferable cut-off value for diagnosing AD was determined to be 10.6 pg/ml. A preferable cut-off value for diagnosing CJD was determined to be 39.8 pg/ml.

According to one preferred embodiment of the present invention, the cut-off values are to be used for a determination by the MRM method using as calibration standard a quantification of an exact protein concentration of a βSyn stock solution by amino acid analysis, more preferably the cut-off values are to be used for a determination by the MRM method as described herein.

According to one embodiment of the present invention, the determination of the concentration of β-synuclein involves single-molecule arrays (Simoa), preferably the determination of the concentration of β-synuclein is carried out by means of single-molecule arrays, more preferably a step of immunoprecipitation of β-synuclein is carried out before determination of β-synuclein concentration via single-molecule arrays (Simoa).

The single-molecule array technology platform described above is commercially available and marketed under the tradename of *SiMoA*^{™} by Quanterix Corporation, Lexington, Mass., USA at the time of filing. However, other versions or modifications of such single-molecule arrays or technology platforms may alternatively be used accordingly. In this specific preferred embodiment, it is more preferred that the setup of the single-molecule array employs a monoclonal anti β-synuclein antibody coupled to carboxylated paramagnetic beads and/or a biotinylated monoclonal anti α- and β-synuclein antibody as a detection antibody and/or streptavidin-β-galactosidase (SBG) as enzyme reagent and/or Resorufin β-D Galactopyranoside as enzyme substrate.

According to one embodiment of the present invention, it is preferred to carry out a step of immunoprecipitation of β-synuclein prior to determining the concentration of β-synuclein in a sample of a patient.

According to the second aspect of the present invention, the use of β-synuclein as a biomarker is provided for diagnosing or assessing the status of a disease or diseases associated with synaptic degeneration as defined in the claims. The use of β-synuclein should not be understood to be limited as long as it is suitable for diagnosing or assessing the status of a disease or diseases associated with synaptic degeneration.

Within this context, the use of β-synuclein as a biomarker is applicable in all types and steps of determination of disease parameters and indications which may contribute to or be considered as a "diagnosing" or "assessing the status of a disease or diseases". Also, the preferred embodiments of the method of the present invention as described hereinabove should be understood to also relate to the use of the invention, if deemed technically sensible to a skilled person

In particular, the use of the present invention is preferably for predicting and/or evaluating response to therapy of a patient diagnosed with or suspected of having a disease or diseases associated with synaptic degeneration. Also preferably, it is for classifying a stage of a patient diagnosed with or suspected of having a disease or diseases associated with synaptic degeneration. Further preferably, it is for selecting the mode of treatment of a patient diagnosed with or suspected of having a disease or diseases associated with synaptic degeneration. It is also preferable for monitoring disease control of a patient diagnosed with or suspected of having a disease or diseases associated with synaptic degeneration. Preferably, it is for monitoring disease progression of a patient diagnosed with or suspected of having a disease or diseases associated with synaptic degeneration.

According to one preferred embodiment of the second aspect of the present invention, β-synuclein is used as biomarker in serum of a patient.

In the present invention, the inventors successfully established an MRM method for the quantification of the synaptic marker protein βSyn in human serum in the very low pg/mL range. Increased βSyn levels were shown in CSF and serum of AD patients. βSyn is already increased in the early disease phase of AD (AD-MCI) and was also increased in CJD but not bvFTD and ALS.

The results presented herein are in agreement with studies of other synaptic proteins in CSF such as neurogranin, SNAP-25 or GAP-43 showing increased levels in AD due to release from degenerating synapses. CSF βSyn concentration already peaked in AD-MCI patients (similar to neurogranin) representing the prodromal phase of AD which is in line with an early involvement of synaptic degeneration in AD pathogenesis.

More importantly, increased βSyn concentrations were also observed in serum of AD patients and showed a similar diagnostic power compared with CSF βSyn. This is of high clinical relevance since no synaptic marker in blood is available so far. Here, the advantage of βSyn might be its more specific expression in the CNS. In addition, presynaptic markers are more affected than postsynaptic markers in AD brains which is why concentration differences of the presynaptic βSyn might be more pronounced in blood. Other synaptic proteins have not been measured in blood so far.

Synaptic dysfunction might be a valuable read-out for clinical drug trials due to its importance in AD pathogenesis and correlation with clinical symptoms. Blood collection is faster, more cost effective and more convenient for patients than CSF collection which is why serum βSyn measurement is advantageous to CSF βSyn or other synaptic CSF markers. It might enable a more close-meshed monitoring of synaptic degeneration in longitudinal studies and the screening of larger populations for patient selection in clinical trials.

Because AD is often associated with comorbidities in the clinic, the strategy in the biomarker field changed in the last years from looking for a single diagnostic biomarker for a clinical syndrome to the discovery of biomarkers reflecting the underlying pathologies. This is important to enable personalized medicine and also defines clearer targets for drug development. In this context, the ATN classification ("A"myloid pathology, "T"au pathology, "N"eurodegeneration) of biomarkers was established in AD including the CSF markers Aβ42 ("A"-group), phosphorylated tau ("T"-group) and total tau ("N"-group).

Markers of synaptic degeneration are assigned to the "N"-group and the present invention provides evidence that βSyn is a reliable synaptic marker which can be added to this panel. To date, only CSF markers are included in the ATN panel of fluid biomarkers but the recent advances in sensitivity of assays for CNS-derived proteins will enable the addition of blood markers in future. Assays for Aβ and tau determination in blood are already available and βSyn is the first synaptic blood marker which can be added, enabling ATN classification also in blood.

βSyn levels were also analyzed in bvFTD and CJD patients since they are relevant in the differential diagnosis of AD. BvFTD patients did not show a significant alteration of βSyn in CSF and serum which is in agreement with studies on other synaptic CSF markers. In contrast, CJD patients showed a massive increase of βSyn in CSF and serum and could discriminate CJD from AD and the other diseases with nearly 100% sensitivity and specificity.

This observation is in agreement with synaptic degeneration as a primary neuropathological feature of CJD and the synaptic location of the prion protein whose misfolding drives the degenerative process in CJD. The synaptic involvement in CJD might also be reflected in the stronger increase of βSyn concentration (30x) compared with tau concentration (8x) in the present invention since tau is thought to be a more general marker of neurodegeneration.

Thus, βSyn is an ideal diagnostic biomarker for CJD in blood. Another candidate is the neurofilament light chain (NfL) but it is also increased in ALS. βSyn in not changed in ALS and therefore, it is a more suitable blood marker for CJD diagnosis than NfL. The unchanged serum βSyn levels in ALS also indicate that βSyn is not just a general marker of neurodegeneration.

In addition to AD and CJD, a large potential for serum βSyn measurement may be present in other indications. Synaptic dysfunction and degeneration is associated with many other neurological diseases or insults of the CNS such as traumatic brain injury, stroke or alcoholism where a synaptic marker in blood might be useful for diagnosis, prognosis, personalized treatment or drug development.

In conclusion, the present data provides βSyn as a blood marker for synaptic degeneration in AD which might be used in clinical trials, patient follow-up and personalized treatment. Serum βSyn is ideally suited as a diagnostic marker for CJD with nearly 100% sensitivity and specificity and might be a useful biomarker in several other neurological indications such as stroke and traumatic brain injury.

All embodiments of the present invention as described herein are deemed to be combinable in any combination, unless the skilled person considers such a combination to not make any technical sense.

### Examples

### 1. MRM Analyses

### Patients

Patients of the discovery cohort were recruited at the Department of Neurology, Ulm University Hospital with additional CJD patients from the surveillance unit for transmissible spongiform encephalopathies at the Department of Neurology in Göttingen. The validation cohorts were recruited independently at different clinical centers in Germany. The technician/scientist performing the βSyn analyses were blinded to the diagnoses of patients in the validation cohorts. Demographic data of the patients are listed in table 1.

**Table 1. Demographic data of patients**

| Patients | N (f/m)¹ | Age (yr)¹ | CSF βSyn (pg/mL)¹ | Ser βSyn (pg/mL)¹ | CSF Tau (pg/mL)¹ | CSF Aβ42 (pg/mL)¹ | MMSE¹ | CDR-SB/CDR¹ |
|---|---|---|---|---|---|---|---|---|
| *Discovery cohort (Ulm)* | | | | | | | | CDR-SB |
| Con | 45 (24/21) | 66 (60-77) | 659 (521-860)² | 8.9 (7.1-10.4)² | 303 (254-381)² | 1486 (1137-1679)² | n.a. | n.a. |
| AD | 56 (38/18) | 73 (68-78) | 979 (738-1223) | 12.9 (9.8-16.4)² | 749 (576-977) | 470.5 (415-565) | 23 (18-26)² | 3.5 (2.0-5.0) |
| bvFTD | 16 (7/9) | 66 (60-69) | 770 (635-975)² | 8.0 (6.1-11.7)² | 361 (303-497) | 883 (733-1192) | n.a. | n.a. |
| ALS | 30 (13/17) | 65 (57-70) | 674 (531-968) | 8.8 (7.1-10.3)² | 365 (285-472) | 1295 (890-1490) | n.a. | n.a. |
| CJD | 25 (16/9) | 64 (59-69) | 19324 (10560-42570) | 479 (259-843) | 2294 (2283-3259)² | n.a. | n.a. | n.a. |
| p-value³ | - | <0.001⁴ | <0.001⁵ | <0.001⁶ | <0.001⁷ | <0.001⁸ | - | - |

| | | | | | | | | |
|---|---|---|---|---|---|---|---|---|
| AD, Alzheimer's disease; ALS, amyotrophic lateral sclerosis; bvFTD, behavioral variant of frontotemporal dementia; CDR, clinical dementia rating; CDR-SB, CDR sum-of-boxes; CJD, Creutzfeldt-Jakob disease; Con, non-neurodegenerative or disease control patients; CSF, cerebrospinal fluid; f, female; m, male; MMSE, Mini Mental State Examination; n.a., not available; Ser, serum; yr, years ¹Data are median and interquartile range ²not available for all patients ³Kruskal-Wallis Test and Dunn's post hoc test ⁴p<0.05: bvFTD vs. AD; p<0.001: ALS vs. AD, CJD vs. AD ⁵p<0.001: Con vs. AD, Con vs. CJD, CJD vs. ALS, CJD vs. AD ⁶p<0.01: Con vs. AD; p<0.001: Con vs. CJD, CJD vs. ALS, CJD vs. AD ⁷p<0.001: AD vs. Con, ALS and bvFTD, CJD vs. Con, ALS and bvFTD ⁸p<0.001: AD vs. Con, ALS and bvFTD | | | | | | | | |

AD was diagnosed according to the NIA-AA criteria (McKhann, GM et al. (2011) Alzheimer's Dement. 7, 263-269), the behavioral variant of frontotemporal dementia (bvFTD) according to Rascovsky *et al.* (Rascovsky, K. et al. (2011) Brain 134, 2456-77), amyotrophic lateral sclerosis according to Ludolph *et al.* (Ludolph, A. et al. (2015) Amyotroph. Lateral Scler. Frontotemporal Degener. 16, 291-2) and Creutzfeldt-Jakob disease patients were neuropathologically verified (World Health Organization. Consensus on criteria for sporadic CJD, 1998). Patients in the control groups had no neurodegenerative disease (Ulm).

Diagnoses of control patients included: Ulm cohort: Control patients from Ulm were patients who received a lumbar puncture to exclude an inflammation of the central nervous system (facial palsy (n=19), tension headache (n=9), arteritis of temporal artery (n=3), migraine (n=2), neuropathia vestibularis (n=2) and one of each diagnoses: cortison induced manic disorder, neuralgia of the pudendus nerve, polymyalgia rheumatica, intoxication, hemispasm of the facial nerve, vertigo, Meige syndrom, L5-syndrom, neuropathia of optic nerve, panic disorder.

All patients or their legal representatives gave written informed consent to be included in the study and the Ethics Committees of the participating clinical centers approved the study.

CSF, serum and clinical scores were collected during diagnostic workup of patients. CSF was collected by lumbar puncture according to local SOPs, centrifuged and stored within 2h at - 80°C. Serum was treated likewise. The inventors used Mini Mental State Examination (MMSE) and clinical dementia rating (CDR) to estimate the severity of dementia and cognitive impairment. The CDR is given as sum-of-boxes (CDR-SB) in the Ulm cohort. The inventors used established CDR-SB cut-offs for subgrouping of AD patients into patients with mild cognitive impairment due to AD (AD-MCI, CDR-SB<2.5) and demented AD patients (CDR-SB≥2.5) (O'Bryant, SE et al. (2010) Arch. Neurol. 67, 746-9).

### Reagents

Recombinant full-length βSyn (#S-1003) and ¹⁵N-labelled full-length βSyn (custom synthesis) were purchased from rPeptide (Bogart, GA, USA). The proteins were dissolved in water and the exact protein concentration of the βSyn stock solution was quantified using amino acid analysis (Alphalyse A/S, Odense, Denmark). The monoclonal anti-βSyn antibody EP1537Y was purchased from Abcam (Cambridge, UK).

### Calibration standards

Calibration standards for CSF measurements were prepared in artificial CSF (aCSF, EcoCyte Bioscience, Austin, TX, USA) containing 200µg/mL human serum albumin with the following βSyn concentrations: 21.8, 43.6, 87.1, 218, 305, 436, 871, 2178, 3485 and 4356pg/mL. For serum measurements, calibration standards were prepared in PBS containing 1% serum with the following βSyn concentrations: 2.0, 4.0, 6.0, 8.0, 10.0, 15.0, 20.0 and 40.0pg/mL.

### Sample preparation for CSF-MRM of βSyn

CSF samples and calibration standards (200µL each) for βSyn MRM analysis were prepared as described previously (Oeckl, P. et al. (2016) Mol. Cell. Proteomics. 15, 3126-3138) but using ¹⁵N-labelled full-length βSyn as internal standard (IS). In brief, samples were spiked with TEAB buffer, ¹⁵N-βSyn and trypsin/LysC and digested for 16h at 27°C. Digestion was stopped with TFA (1% final) and fractionated by centrifugation through strong cation exchange STAGE-Tips into 3 fractions. The fractions were stored in a cooled autosampler until analysis.

### Sample preparation for serum IP-MRM of βSyn

The EP1537Y anti-βSyn antibody was covalently coupled to epoxy-coated magnetic beads (Dynabeads Antibody Coupling Kit, #14311D, Thermo, Waltham, MA, USA) for 20-24h at 37°C according to the manufacturer's instructions using 2µg antibody per mg bead. The EP1537Y-coated beads were washed with 100mM glycine-HCl (pH 2.5)/0.05% Tween and finally resuspended in PBS/0.05% Tween until use.

Serum samples and calibration standards (950µL each) were mixed with 135µL internal standard solution (815mM TEAB, 0.37% Tween, 1.78ng/mL ¹⁵N-βSyn, final concentration: 100mM TEAB, 0.05% Tween, 22.2pg/mL ¹⁵N-βSyn) in protein low-binding tubes (Sarstedt, Nürnbrecht, Germany), that were coated with NaCl. The samples were pre-cleared using 0.5mg uncoupled beads for 1h at room temperature followed by immunoprecipitation of βSyn with 0.5mg EP1537Y-coated beads per sample for 20-24h at 4°C on a rotator.

The beads were washed three times with 500µL 100mM TEAB and eluted with 25µL 50mM glycine-HCl (pH 2.5). Protein digestion was performed for 2.5h at 37°C by adding 10µL of 50ng/mL trypsin/LysC (Promega, Madison, WI, USA) (dissolved in 500mM TEAB) and stopped using 5µL 2% TFA/72%acetonitrile. Samples were stored in a cooled autosampler until analysis.

### LC-MS/MS analysis and MRM settings

βSyn MRM analysis was performed with an QTRAP6500 mass spectrometer coupled to an Eksigent MicroLC200 (both AB Sciex, Darmstadt, Germany), 1260 HPLC pump (Agilent, Santa Clara, CA, USA) and cooled HTC PAL-xt autosampler (CTC Analytics AG, Zwingen, Switzerland). For liquid chromatography a C18 PepMap100, 5µm, 0.3x5.0mm trap column (Thermo, Waltham, MA, USA) and Eksigent HALO Fused-core C18, 2.7µm, 0.5x100 mm analytical column were used. Two proteotypic peptides of βSyn were quantified by MRM (βSyn46-58 and βSyn61-85, see Fig. 1A and table 2). Chromatographic conditions and MRM settings are described by Oeckl *et al.,* 2016 (*loc. cit.*)*.*

**Table 2. βSyn tryptic peptides used for MRM analysis**

| βSyn tryptic peptide | Precursor mass (m/z) | Charge state (z) | Fragment mass (m/z) | CE (V) |
|---|---|---|---|---|
| βSyn46-58 (EGVVQGVASVAEK) | 636.8 (light) | 2 | 760.4 (y8+) | 31.0 |
| | | | 888.5 (y9+) | 30.2 |
| | | | 987.6 (y10+) | 31.5 |
| | 644.3 (heavy) | 2 | 769.4 (y8+) | 31.0 |
| | | | 899.5 (y9+) | 30.2 |
| | | | 999.5 (y10+) | 31.5 |
| βSyn61-85 (EQASHLGGAVFSGAGNIAAATGLVK) | 776.1 (light) | 3 | 730.5 (y8+) | 33.5 |
| | | | 798.9 (b17++) | 27.5 |
| | | | 659.4 (y7+) | 35.0 |
| | 786.1 (heavy) | 3 | 739.4 (y8+) | 33.5 |
| | | | 809.4 (b17++) | 27.5 |
| | | | 667.4 (y7+) | 35.0 |

| | | | | |
|---|---|---|---|---|
| CE, collision energy | | | | |

An external calibration curve was used for quantification using the peak area ratio (light/heavy) and a weighting of 1/x². Linear regression was used for CSF measurements and a quadratic curve for serum IP-MRM. Data were analyzed using Analyst Software 1.6.2 (AB Sciex, Darmstadt, Germany) and Skyline 4.1 (MacLean, B. *et al.* (2010) *Bioinformatics* **26,** 966-8). To calculate βSyn concentration in CSF, the inventors used the mean concentration of both measured peptides.

In serum, the inventors used the peptide βSyn46-58 as the quantifier and βSyn61-85 as qualifier, since βSyn61-85 was often near the limit of detection and showed higher variability. CSF and serum quality control (QC) samples were included in all runs to monitor intra- and inter-assay precision and accuracy.

### Determination of Tau and Aβ42

The core AD biomarkers Tau and Aβ42 were determined in CSF by ELISAs from Fujirebio (Gent, Belgium) according to the manufacturer's instructions.

### Statistics

Statistical analysis was performed by GraphPad Prism 5.00. Group comparisons were made using two-tailed Mann-Whitney test (two groups) or Kruskal-Wallis test and Dunn's post hoc test (>2 groups). Correlation analysis was performed using Spearman's rank correlation coefficient.

A linear regression model was used for age-adjustment of CSF and serum βSyn values for group comparisons. The diagnostic potential of βSyn was calculated using receiver operating characteristic (ROC) curve analysis and diagnostic cut-offs were selected using the Youden index. A p-value <0.05 was regarded as significant.

### Results

The inventors successfully established a method for the absolute quantification of the presynaptic protein βSyn in serum by mass spectrometry. Representative chromatograms of the two measured peptides are shown in Fig. 1B and the measured values of both peptides strongly correlated (r=0.73, Fig. 1C) supporting the specificity of the MRM. The lower limit of quantification (LLOQ) was 2.0pg/mL and intra- and interassay CVs were 3.3-8.6% and 11.7%.

Deviations of diluted samples (up to fourfold) and spike-in samples (+10pg/mL βSyn) were in the desired range of ≤15% and serum βSyn was stable for at least five freeze/thaw cycles and 2h at room temperature. Spike-in of 0.5% whole blood into serum and shock freezing (mimicking hemolysis) did not affect the measurements. This indicates that βSyn in serum is a robust biomarker.

In addition, the previously described βSyn MRM method for CSF (Oeckl *et al.,* 2016; *loc. cit.*) was improved by including the ¹⁵N-labeled full-length βSyn as IS, the gold standard for absolute quantification by MRM (Fig. 1B,C). Intra- and interassay CVs were 0.6-9.3% and 5.1%, dilution stability was tested and confirmed up to fourfold, spike-in CSF samples showed a deviation in the desired range (≤15%) and βSyn was stable in CSF for at least five freeze/thaw cycles.

Using established and validated MRM methods, the inventors investigated CSF and serum βSyn in a well-characterized discovery cohort of AD (n=56) and non-neurodegenerative control patients (Con, n=45) with clear clinical diagnosis (Ulm cohort). The cohort also included other neurodegenerative diseases, i.e. bvFTD (n=16), CJD (n=25) and ALS (n=30). CJD and bvFTD were included since they are relevant in the differential diagnosis of AD, and ALS served as a disease characterized by rapid and severe neurodegeneration for comparison with CJD.

Serum βSyn concentration in controls was about 75x lower than in CSF (median 8.9pg/mL vs. 659pg/mL). This difference is in the expected range known from other CNS-derived proteins. The inventors observed a very weak (CSF, r=0.23) and weak (serum, r=0.32) but significant correlation of βSyn concentration with age in control patients (p<0.05 for CSF, p<0.01 for serum). Therefore, age was included as confounding factor in statistical tests but not for calculation of ROC curves and cut-off values.

There was no gender effect on βSyn concentration in CSF (p≥0.43) by comparing female and male patients within all groups. Serum βSyn was also not different in male and female patients in the Ulm cohort (p=0.64 in Con and p=0.39 in AD).

Significantly increased CSF βSyn concentrations were observed in AD patients (p<0.001) but not bvFTD and ALS patients compared with controls in the discovery cohort (Fig. 2A and table 1). In addition, a large increase (30-fold) of CSF βSyn was observed in CJD compared to all other groups (p<0.001).

There was a similar pattern of βSyn concentration in serum compared with CSF. The inventors observed significantly increased serum βSyn in AD (p<0.01) and more pronounced in CJD patients (p<0.001) compared with controls in the discovery cohort (Fig. 2B and table 1).

The AD group of the Ulm cohort was divided into AD-MCI (CDR-SB<2.5) and demented AD patients (CDR-SB≥2.5). CSF βSyn was already significantly increased in AD-MCI patients (p<0.01) and also in demented AD patients (p<0.001) compared with controls (Fig. 3). Serum βSyn was increased in demented AD patients (p<0.001) and did not reach the significance level in MCI patients (Fig. 3).

CSF βSyn showed a moderate correlation with serum βSyn (r=0.55, p<0.001, Fig. 4A) and a strong correlation with CSF Tau (r=0.81, p<0.001, Fig. 4A) in the Ulm cohort. Correlation of CSF βSyn with CSF Aβ42 was weak (r=-0.34, p<0.001). Serum βSyn also showed a moderate correlation with CSF Tau (r=0.53, p<0.001). There was no correlation of CSF and serum βSyn with the CDR-SB in AD patients as a measure of dementia severity (r=-0.16, p=0.24 and r=0.26, p=0.09).

The correlation of CSF βSyn with the MMSE was slightly significant in AD patients of the Ulm cohort (r=0.31, p=0.03). ROC curve analysis was used to test the diagnostic potential of βSyn. CSF βSyn showed a sensitivity and specificity of 91% and 54% for AD vs. controls at a cut-off of 685pg/mL in the Ulm cohort (n=99) and 77% and 66% at a cut-off of 686pg/mL in the cumulative cohort including data from additional centers (data from additional centers not shown individually; n=255, Fig. 4B, table 3).

The diagnostic power of serum βSyn was similar to CSF with a sensitivity and specificity of 75% and 78% in the Ulm cohort (cut-off 10.6pg/mL, n=76) and 74% and 69% in the cumulative cohort (cut-off 10.2pg/mL, n=221, Fig. 4B, table 3). Notably, both CSF and serum βSyn discriminated CJD from controls, AD and ALS with nearly 100% sensitivity and specificity (table 3).

**Table 3. ROC curve analysis of CSF and serum βSyn concentration**

| Comparison | Biomarker | Sensitivity % (95%CI) | Specificity % (95%CI) | AUC (95%CI) | Cut-off (pg/mL) |
|---|---|---|---|---|---|
| *Ulm cohort* | | | | | |
| AD vs. Con (n=99) | CSF βSyn | 91 (80-97) | 54 (38-69) | 0.76 (0.66-0.86) | 685 |
| AD vs. bvFTD (n=71) | CSF βSyn | 61 (47-74) | 73 (45-92) | 0.69 (0.53-0.84) | 891 |
| AD vs. CJD (n=81) | CSF βSyn | 100 (86-100) | 98 (91-100) | 1.00 (1.00-1.00) | 2153 |
| CJD vs. Con (n=68) | CSF βSyn | 100 (86-100) | 98 (88-100) | 1.00 (1.00-1.00) | 1836 |
| CJD vs. ALS (n=55) | CSF βSyn | 100 (86-100) | 100 (88-100) | 1.00 (1.00-1.00) | 1881 |
| AD vs. Con (n=76) | Ser βSyn | 75 (60-87) | 78 (60-91) | 0.75 (0.64-0.86) | 10.6 |
| AD vs. bvFTD (n=54) | Ser βSyn | 90 (56-100) | 57 (41-72) | 0.76 (0.61-0.92) | 12.0 |
| AD vs. CJD (n=69) | Ser βSyn | 96 (80-100) | 100 (92-100) | 0.98 (0.94-1.02) | 46.0 |
| CJD vs. Con (n=57) | Ser βSyn | 96 (80-100) | 100 (89-100) | 0.99 (0.98-1.01) | 39.8 |
| CJD vs. ALS (n=54) | Ser βSyn | 96 (80-100) | 100 (88-100) | 1.00 (0.99-1.01) | 37.8 |

| *Cumulative cohort* | | | | | |
|---|---|---|---|---|---|
| AD vs. Con (n=255) | CSF βSyn | 77 (70-84) | 66 (56-74) | 0.76 (0.71-0.82) | 686 |
| AD vs. Con (n=221) | Ser βSyn | 74 (66-82) | 69 (58-78) | 0.73 (0.67-0.80) | 10.2 |

| | | | | | |
|---|---|---|---|---|---|
| AD, Alzheimer's disease; ALS, amyotrophic lateral sclerosis; AUC, area under the ROC curve; bvFTD, behavioral variant of frontotemporal dementia; CJD, Creutzfeldt-Jakob disease; Con, non-neurodegenerative or disease control patients; CSF, cerebrospinal fluid; Ser, serum | | | | | |

### 2. SiMoA Analyses

### Patients

All samples (CSF and serum) examined in this study were taken from patients seen in the Departments of Neurology Ulm and Göttingen. Collection and analysis of serum specimen was approved by the Ethics Committee in Ulm (approval number 20/10) and Göttingen (100305). All patients signed an informed consent previous to study enrolment and underwent clinical, neurological, and partly neuroradiological examinations. All methods were carried out in accordance with the approved guidelines.

Patients from Ulm were divided into 6 groups according to their diagnosis; Alzheimer's Disease (AD), Amyotrophic lateral sclerosis (ALS), behavioral variant frontotemporal dementia (bvFTD), Synucleinopathies (Parkinson's disease (PD), Dementia with Lewy bodies (DLB), Parkinson's disease dementia (PDD)), Creutzfeldt-Jakob disease (CJD) and non-demented controls (NDC).

The diagnosis of 70 AD patients from Ulm was made according to the International Working Group 2 criteria (Dubois B. et al. (2014) The Lancet Neurology, 13, 614-629). The 29 ALS patients were diagnosed with definite or probable ALS according to the revised El Escorial criteria (Brooks BR. et al. (2000) Amyotrophic lateral sclerosis and other motor neuron disorders: official publication of the World Federation of Neurology, Research Group on Motor Neuron Diseases, 1, 293-299). bvFTD patients (n=18) were diagnosed according to the international criteria (Rascovsky *et al.,* 2011; *loc. cit.*)*.*

46 synucleinopathy subjects were analyzed which were diagnosed by specialists for movement disorders according to the United Kingdom PD Society Brain Bank criteria (Hughes AJ et al. (1992) Journal of neurology, neurosurgery, and psychiatry, 55, 181-184). All 23 CJD patients were neuropathologically confirmed cases analyzed in the unit for transmissible spongiform encephalopathies of the Department of Neurology in Göttingen (Brown P et al. (2003) WHO Manual for Surveillance of Human Transmissible Spongiform Encephalopathies Including Variant Creutzfeldt-Jakob Disease. World Health Organization, Communicable Disease Surveillance and Response).

In the non-demented control cohort, 65 subjects were included from Ulm without clinical and radiological signs for neurodegeneration (e.g. subjective complaints, tension headache, facial nerve paralysis (non-inflammatory)). Furthermore, all non-demented control patients presented without being positive for all three AD typical markers (total Tau >450 pg/ml, phospho-Tau (p-Tau) >61 pg/ml and Aβ42 <550 pg/ml in the CSF).

The stratification of the AD cohort into patients with AD and AD with only mild cognitive impairments (MCI) was done according to the clinical dementia rating (CDR) scale (Hughes CP et al. (1982) The British journal of psychiatry: the journal of mental science, 140, 566-572). For the evaluation the CDR sum of boxes (CDR SOB) was chosen (O'Bryant, SE et al. (2008) Archives of neurology, 65, 1091-1095). Patients presenting with a CDR SOB below 2.5 were classified as AD-MCI (O'Bryant, SE *et al.,* 2010; *loc. cit.*)*.* The diagnosis of PD-MCI was made according to consensus criteria proposed by the Movement Disorder Society task force (Litvan I et al. (2012) Diagnostic criteria for mild cognitive impairment in Parkinson's disease: Movement Disorder Society Task Force guidelines. Movement disorders: official journal of the Movement Disorder Society, 27, 349-356).

### Laboratory markers and assay reproducibility

Serum samples were received from peripheral blood by centrifugation (800 g, 5 min), aliquoted and stored within 2 hours at -80°C until analysis. Serum examinations included serum neurofilament light chain (NfL) and beta-synuclein both measured with a Simoa platform (Quanterix, Lexington, MA, USA). For NfL analysis the commercially available NfL kit from Quanterix was applied. For the measurement of beta-synuclein an in-house homebrew assay was established. Serum beta-synuclein assay variability was tested by control samples as triplicates in two different runs. For serum beta-synuclein analysis, the intra-assay variability was determined to be 4.3% and the inter-assay 12.8%. All values are to be understood as mean values. Samples were stable after up to five freeze and thaw cycles as well as for 2h storage at room temperature (variability <6%).

### Beta-synuclein serum Simoa assay in detail

For the analysis of serum beta-synuclein, a highly sensitive "homebrew" assay was established for the Simoa platform from Quanterix. Therefor, the monoclonal anti beta-synuclein specific EP1537Y antibody from Abcam was coupled to carboxylated paramagnetic beads (Quanterix, Lexington, MA, USA, 100451) according to the protocol of the manufacturer. Furthermore, the monoclonal anti alpha- and beta-synuclein antibody EP1646Y free of bovine serum albumin and azide from Abcam (ab189217) was biotinylated with 40x molar excess of biotin and subsequently used as detection antibody.

For biotinylation the EZ-Link^{®} Micro Sulfo-NHS-Biotinylation Kit (Thermo #21925) was used. Buffers for beads and detector were PBS-Tween 0.5% and PBS-Tween 0.05%, respectively. Streptavidin-β-galactosidase (SBG) provided by Quanterix (100439) was diluted in SBG buffer provided by the manufacturer (100376) to a final concentration of 150 pM.

As substrate, Resorufin β-D-Galactopyranoside (RGP) (Quanterix, 103159) was used. Calibrators ranging from 0.625 to 100 pg/ml were prepared in sodium chloride (NaCl) (2M) with recombinant beta-synuclein (rPeptide, S-1003-1) and 400 µl of each was added to a 96-well plate provided by Quanterix. Serum samples were diluted one to one with 4M NaCl resulting in a final concentration of 2M NaCL and shaken for 10 min at RT at 1200 rpm. 220 µl of diluted sample were added to each well. Beads, Detector, SBG, Plate and substrate were placed into the Simoa platform and the custom assay started.

Crucial for the successful establishment of this assay are the following steps/reagents
- two Abcam antibodies mentioned above
- sample/calibrator preparation using a self-made sample/calibrator buffer (4M/2M NaCl)
- extended beads/sample incubation time of 120 min (standard 15 min)
- use of a self-made bead buffer with an uncommonly high concentration of Tween (0.5%)
- high Volume of 172 µl of sample/calibrator (standard 100 µl)

### Statistical analysis

Mann-Whitney U test was applied to determine significant differences in two groups. For comparisons of three or more groups Kruskal-Wallis test with subsequent Dunn post hoc test in case of significant results, was chosen. For cut-off calculations receiver operating characteristics (ROC) analyses were performed. Cut-off levels were selected for maximizing the Youden Index (sensitivity+specificity-1) and the best likelihood ratio. To determine significant correlations between parameters the Spearman rank correlation coefficient was used. For all analyses p<0.05 was considered statistically significant. Statistical calculations were performed applying the GraphPad Prism 5.0 software (GraphPad Software, La Jolla, CA, USA).

### Demographic and clinical features of the Ulm cohort

All relevant demographic and clinical parameters are summarized in table 4. Age did not differ significantly between groups except for ALS and CJD patients which were younger compared to the AD cohort (p<0.0001).

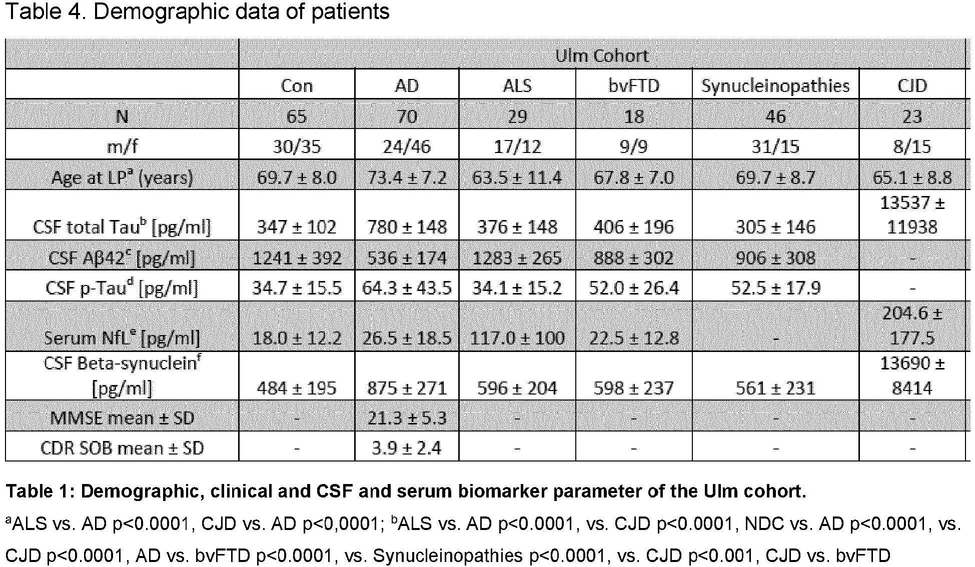

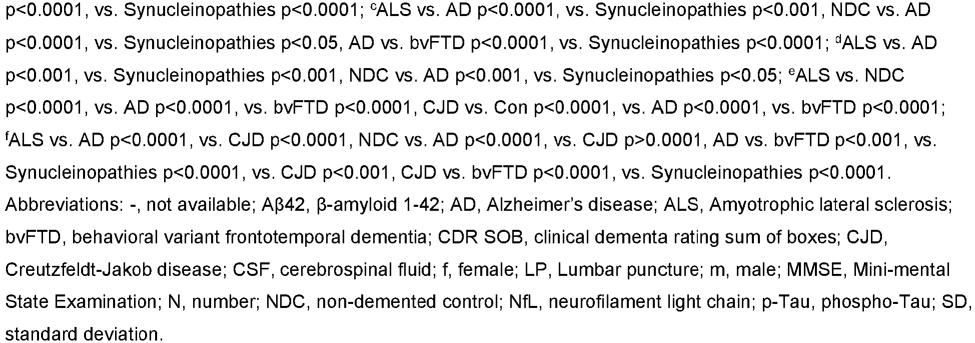

No association was found between serum beta-synuclein levels and age in all patients (r = -0.14 (Cl: -0.32-0.05), p=0.14) as well as in no individual cohort. There was no significant difference in gender between the cohorts except for PD vs. NDC/CJD/AD and AD vs. ALS. However, sex had no effect on CSF and serum beta-synuclein levels (data not shown). The median Mini-mental State Examination (MMSE) and CDR SOB values in the AD cohort were 23 and 3.25 respectively.

### Establishment of a Simoa assay for the detection of beta-synuclein in blood

For the measurement of beta-synuclein, the inventors developed a sensitive Simoa assay for the analysis of blood beta-synuclein with an intra- and interassay cv of 4.3% and 12.8%, respectively. The Simoa results demonstrate high associations to the blood beta-synuclein levels measured by mass spectrometry (r = 0.76 (Cl: 0.65-0.84), p<0.0001) (Fig. 5).

### Serum beta-synuclein quantitation

For the analysis of beta-synuclein in serum, it was necessary to establish a highly sensitive Simoa approach due to the low concentration of the synaptic protein in blood. After successful development of the Simoa, the inventors analyzed in total 112 serum samples from Ulm grouped into 35 NDC, 39 AD, 16 bvFTD and 22 CJD patients (see Fig. 6A).

CJD patients showed the highest beta-synuclein levels which were statistically significantly increased (p<0.0001) compared to the other three cohorts. Between AD and NDC, elevated beta-synuclein levels could be detected in AD subjects (see Fig. 6B). There was no difference in serum beta-synuclein values between AD and bvFTD patients.

### Immunoprecipitation of beta-synuclein

To increase sensitivity further, the inventors performed an immunoprecipitation (IP) of beta-synuclein in serum before the analysis via Simoa. For the validation of the IP Simoa, 17 NDC, 25 AD, 4 CJD and this time also ALS patients were chosen (n=21). In the IP measurement, the median serum beta-synuclein levels of the CJD patients were not only significantly elevated (p<0.0001) compared to NDC patients but showed also higher levels (p<0.05) than the ALS subjects. AD cases displayed, comparable to the Simoa approach without previous IP, increased levels (p<0.05) compared to controls (see Fig. 7A).

### Serum NfL levels and ROC analysis

In addition to the evaluation of serum beta-synuclein, the inventors also analyzed serum NfL levels in the patient cohorts with the aim to compare them to the serum beta-synuclein levels (see Fig. 7B). As already described ALS NfL levels were significantly higher (p<0.0001) than AD, bvFTD and NDC levels (AD: 21.3, bvFTD: 20.5, NDC: 15.2 pg/ml). However, a significant difference between ALS and CJD (139 pg/ml) patients could not be detected.

This finding prompted us to analyze beta-synuclein in the serum of a higher number of ALS and CJD patients as well as in a cohort of subjects with a "false positive" NfL result (currently ongoing). Performing ROC analyses of serum beta-synuclein levels of ALS/AD/NDC vs. CJD patients, nearly perfect discriminations were found in all ROC curves (preliminary results, see Fig. 7C).

For the evaluation of beta-synuclein in blood, the inventors developed an ultrasensitive Simoa, hoping to assess the synaptic protein also in the serum of patients which would be of great value in longitudinal neurological studies and clinical trials. Serum beta-synuclein results show that also in blood CJD patients have the by far highest values and the AD levels seem also to be increased compared to the NDC cohort albeit to a much smaller degree than in CSF. Reasons for the large overlap with control patients can be manifold and allocated into two groups; (i) physiological reasons, (ii) assay issues.

To (i), beta-synuclein seems to be almost exclusively expressed in the brain. (ii), cross reactivity of beta-synuclein with alpha- and gamma-synuclein was excluded. Matrix effects could interfere with the analysis, however, experiments with spiked beta-synuclein showed good recovery.

As already mentioned, CJD patients displayed clearly elevated beta-synuclein levels also in blood. This was, due to the nature of the rapid neurodegenerative disease, expected but also a proof of principle for the beta-synuclein serum Simoa. Furthermore, the findings demonstrated that CJD patients also have high NfL levels comparable to those of ALS patients, thus the NfL analysis alone cannot distinguish between ALS and CJD. To date, NfL levels in CSF and serum of ALS patients are a widely accepted biomarker in the diagnosis of ALS.

Most interestingly, ALS subjects had in contrast to CJD patients low serum beta-synuclein values rendering the beta-synuclein serum assay an excellent blood-based biomarker test for the differential diagnosis of ALS vs. CJD. Performing ROC analysis for serum beta-synuclein ALS vs. CJD, the two neurological disorders could be distinguished with nearly 100%.

## Claims

1. Method of diagnosis or assessing the status of a disease or diseases associated with synaptic degeneration, wherein the disease or diseases associated with synaptic degeneration are one or more of Alzheimer's disease, Creutzfeldt-Jakob disease, traumatic brain injury, stroke, and/or glioma, the method comprising determining the concentration of ß-synuclein in a sample of a patient, wherein the sample of the patient is blood, serum, or plasma, and wherein the method is carried out *ex vivo.*

2. Method according to claim 1, wherein the sample of the patient is a blood sample, preferably a blood serum sample.

3. Method according to any of claims 1 or 2, wherein the disease or diseases associated with synaptic degeneration are Alzheimer's disease and/or Creutzfeldt-Jakob disease, preferably wherein the disease associated with synaptic degeneration is Alzheimer's disease.

4. Method according to any of the preceding claims, wherein the method is for assessing the status of the disease or diseases associated with synaptic degeneration, or alternatively a method of classifying a stage, preferably a prognostic stage, of a patient diagnosed with or suspected of having the disease or diseases associated with synaptic degeneration, or alternatively a method of monitoring disease progression in patients diagnosed with or suspected of having the disease or diseases associated with synaptic degeneration.

5. Method according to any of the preceding claims, wherein the determination of the concentration of ß-synuclein involves quantitative mass spectrometry, preferably multiple/selected reaction monitoring (MRM/SRM) or parallel reaction monitoring (PRM), more preferably wherein the determination of the concentration of ß-synuclein is carried out by means of multiple/selected reaction monitoring or parallel reaction monitoring, even more preferably by measuring the peptides of aa 46 to 58 (EGVVQGVASVAEK) and/or 61 to 85 (EQASHLGGAVFSGAGNIAAATGLVK) of ß-synuclein.

6. Method according to any of the preceding claims, wherein the cut-off value for diagnosing Alzheimer's disease is at 10 pg/ml of ß-synuclein in serum, preferably at 10.6 pg/ml.

7. Method according to any of the preceding claims, wherein the cut-off value for diagnosing Creutzfeldt-Jakob disease is at 39.8 pg/ml, preferably as determined by Ser MRM (Youden Index).

8. Method according to any of claims 1 to 4, wherein the determination of the concentration of ß-synuclein involves single-molecule arrays (Simoa), preferably the determination of the concentration of ß-synuclein is carried out by means of single-molecule arrays, more preferably wherein a step of immunoprecipitation of ß-synuclein is carried out before determination of ß-synuclein concentration via single-molecule arrays (Simoa).

9. Method according to Claim 8, wherein the setup of the single-molecule arrays employs a monoclonal anti ß-synuclein antibody coupled to carboxylated paramagnetic beads and/or a biotinylated monoclonal anti α- and ß-synuclein antibody as a detection antibody and/or streptavidin-ß-galactosidase (SBG) as enzyme reagent and/or Resorufin ß-D Galactopyranoside as enzyme Substrate.

10. Method according to any of the preceding claims, wherein the method is used for discriminating Creutzfeldt-Jakob disease, Alzheimer's disease, behavioral variant frontotemporal dementia, and amyotrophic lateral sclerosis from each other, wherein the method combines determination of neurofilament (NfL) and ß-synuclein levels in blood, preferably wherein neurofilament (NfL) levels in blood are increased over a reference value for healthy subjects.

11. Use of ß-synuclein as a biomarker for diagnosing or assessing the status of a disease or diseases associated with synaptic degeneration, preferably for evaluating response to therapy of a patient diagnosed with or suspected of having a disease or diseases associated with synaptic degeneration, and/or for classifying a stage of a patient diagnosed with or suspected of having a disease or diseases associated with synaptic degeneration, and/or for monitoring disease control of a patient diagnosed with or suspected of having a disease or diseases associated with synaptic degeneration, and/or for monitoring disease progression of a patient diagnosed with or suspected of having a disease or diseases associated with synaptic degeneration,
wherein the disease or diseases associated with synaptic degeneration are one or more of Alzheimer's disease, Creutzfeldt-Jakob disease, traumatic brain injury, stroke, and/or glioma,
wherein the use comprises determining the concentration of ß-synuclein in a sample of the patient, wherein the sample of the patient is blood, serum, or plasma, and wherein the use is *ex vivo.*

12. Use according to Claim 11, wherein ß-synuclein is used as biomarker in serum of a patient.

## Patentansprüche

1. Verfahren zur Diagnose oder Bewertung des Status einer Krankheit oder von Krankheiten, die mit synaptischer Degeneration assoziiert sind, wobei die Krankheit oder die Krankheiten, die mit synaptischer Degeneration assoziiert sind, eine oder mehrere von Alzheimer-Krankheit, Creutzfeldt-Jakob-Krankheit, traumatischer Hirnverletzung, Schlaganfall und/oder Gliom sind, wobei das Verfahren die Bestimmung der Konzentration von ß-Synuclein in einer Probe eines Patienten umfasst, wobei die Probe des Patienten Blut, Serum oder Plasma ist, und wobei das Verfahren *ex vivo* durchgeführt wird.

2. Verfahren nach Anspruch 1, wobei die Probe des Patienten eine Blutprobe, vorzugsweise eine Blutserumprobe, ist.

3. Verfahren nach einem der Ansprüche 1 oder 2, wobei die mit synaptischer Degeneration assoziierten Krankheiten die Alzheimer-Krankheit und/oder die Creutzfeldt-Jakob-Krankheit sind, vorzugsweise wobei die mit synaptischer Degeneration assoziierte Krankheit die Alzheimer-Krankheit ist.

4. Verfahren nach einem der vorhergehenden Ansprüche, wobei das Verfahren ein Verfahren zur Bewertung des Status der Krankheit oder der Krankheiten, die mit synaptischer Degeneration assoziiert sind, ist oder alternativ ein Verfahren zur Klassifizierung eines Stadiums, vorzugsweise eines prognostischen Stadiums, eines Patienten, bei dem die Krankheit oder die Krankheiten, die mit synaptischer Degeneration assoziiert sind, diagnostiziert sind oder vermutet werden, oder alternativ ein Verfahren zur Überwachung des Fortschreitens der Krankheit bei Patienten, bei denen die Krankheit oder die Krankheiten, die mit synaptischer Degeneration assoziiert sind, diagnostiziert sind oder vermutet werden.

5. Verfahren nach einem der vorhergehenden Ansprüche, wobei die Bestimmung der Konzentration von ß-Synuclein mittels quantitativer Massenspektrometrie, vorzugsweise mittels "multiple/selected reaction monitoring" (MRM/SRM) oder "parallel reaction monitoring" (PRM) erfolgt, weiter bevorzugt, wobei die Bestimmung der Konzentration von ß-Synuclein mittels "multiple/selected reaction monitoring" oder "parallel reaction monitoring" durchgeführt wird, noch weiter bevorzugt durch Messung der Peptide von AS 46 bis 58 (EGVVQGVASVAEK) und/oder 61 bis 85 (EQASHLGGAVFSGAGNIAAATGLVK) von ß-Synuclein.

6. Verfahren nach einem der vorhergehenden Ansprüche, wobei der Grenzwert für die Diagnose der Alzheimer-Krankheit bei 10 pg/ml ß-Synuclein im Serum liegt, vorzugsweise bei 10,6 pg/ml.

7. Verfahren nach einem der vorhergehenden Ansprüche, wobei der Grenzwert für die Diagnose der Creutzfeldt-Jakob-Krankheit bei 39,8 pg/ml liegt, vorzugsweise bestimmt durch Ser MRM (Youden Index).

8. Verfahren nach einem der Ansprüche 1 bis 4, wobei die Bestimmung der ß-Synuclein-Konzentration Einzelmolekül-Arrays (Simoa) einbezieht, vorzugsweise wird die Bestimmung der ß-Synuclein-Konzentration mittels Einzelmolekül-Arrays durchgeführt, noch bevorzugter wird vor der Bestimmung der ß-Synuclein-Konzentration mittels Einzelmolekül-Arrays (Simoa) ein Schritt der Immunpräzipitation von ß-Synuclein durchgeführt.

9. Verfahren nach Anspruch 8, wobei die Einrichtung der Einzelmolekül-Arrays einen monoklonalen Anti-ß-Synuclein-Antikörper verwendet, der an carboxylierte paramagnetische Beads gekoppelt ist, und/oder einen biotinylierten monoklonalen Anti-α- und ß-Synuclein-Antikörper als Nachweisantikörper und/oder Streptavidin-ß-Galactosidase (SBG) als Enzymreagenz und/oder Resorufin-ß-D-Galactopyranosid als Enzymsubstrat.

10. Verfahren nach einem der vorhergehenden Ansprüche, wobei das Verfahren verwendet wird, um die Creutzfeldt-Jakob-Krankheit, die Alzheimer-Krankheit, die verhaltensbedingte Variante der frontotemporalen Demenz und die amyotrophe Lateralsklerose voneinander zu unterscheiden, wobei das Verfahren die Bestimmung des Neurofilament- (NfL) und des ß-Synuclein-Spiegels im Blut kombiniert, wobei vorzugsweise der Neurofilament- (NfL) Spiegel im Blut gegenüber einem Referenzwert für gesunde Personen erhöht ist.

11. Verwendung von ß-Synuclein als Biomarker zur Diagnose oder Bewertung des Status einer Krankheit oder von Krankheiten, die mit synaptischer Degeneration assoziiert sind, vorzugsweise zur Bewertung des Ansprechens auf eine Therapie bei einem Patienten, bei dem eine Krankheit oder Krankheiten, die mit synaptischer Degeneration assoziiert sind, diagnostiziert sind oder vermutet werden, und/oder zur Klassifizierung eines Stadiums eines Patienten, bei dem eine Krankheit oder Krankheiten, die mit synaptischer Degeneration assoziiert sind, diagnostiziert sind oder vermutet werden, und/oder zur Überwachung des Fortschreitens der Krankheit eines Patienten, die mit synaptischer Degeneration assoziiert sind, diagnostiziert sind oder vermutet werden,
wobei die mit synaptischer Degeneration assoziierte(n) Krankheit(en) eine oder mehrere von Alzheimer-Krankheit, Creutzfeldt-Jakob-Krankheit, traumatischer Hirnverletzung, Schlaganfall und/oder Gliom sind,
wobei die Verwendung die Bestimmung der Konzentration von ß-Synuclein in einer Probe des Patienten umfasst, wobei die Probe des Patienten Blut, Serum oder Plasma ist, und wobei die Verwendung ex vivo erfolgt.

12. Verwendung nach Anspruch 11, wobei ß-Synuclein als Biomarker im Serum eines Patienten verwendet wird.

## Revendications

1. Procédé de diagnostic ou d'évaluation du statut d'une maladie ou de maladies associées à la dégénérescence synaptique, dans lequel la maladie ou les maladies associées à la dégénérescence synaptique sont une ou plusieurs parmi la maladie d'Alzheimer, la maladie de Creutzfeldt-Jakob, les lésions cérébrales traumatiques, un accident vasculaire cérébral et/ou un gliome, le procédé comprenant la détermination de la concentration de β-synucléine dans un échantillon d'un patient, dans lequel l'échantillon du patient est du sang, du sérum ou du plasma, et dans lequel le procédé est mis en oeuvre ex *vivo.*

2. Procédé selon la revendication 1, dans lequel l'échantillon du patient est un échantillon de sang, de préférence un échantillon de sérum sanguin.

3. Procédé selon l'une quelconque des revendications 1 ou 2, dans lequel la maladie ou les maladies associées à la dégénérescence synaptique sont la maladie d'Alzheimer et/ou la maladie de Creutzfeldt-Jakob, de préférence dans lequel la maladie associée à la dégénérescence synaptique est la maladie d'Alzheimer.

4. Procédé selon l'une quelconque des revendications précédentes, dans lequel le procédé est destiné à évaluer le statut de la maladie ou des maladies associées à la dégénérescence synaptique, ou en variante un procédé de classification d'un stade, de préférence un stade pronostique, d'un patient diagnostiqué de ou suspecté d'être atteint de la maladie ou des maladies associées à la dégénérescence synaptique, ou en variante un procédé de surveillance de la progression de la maladie chez des patients diagnostiqués de ou suspectés d'être atteints de la maladie ou des maladies associées à la dégénérescence synaptique.

5. Procédé selon l'une quelconque des revendications précédentes, dans lequel la détermination de la concentration en β-synucléine implique une spectrométrie de masse quantitative, de préférence un suivi de réactions multiples/sélectionnées (MRM/SRM) ou un suivi de réactions parallèles (PRM), plus préférablement dans lequel la détermination de la concentration en β-synucléine est effectuée au moyen d'un suivi de réactions multiples/sélectionnées ou d'un suivi de réactions parallèles, encore plus préférablement en mesurant les peptides des aa 46 à 58 (EGVVQGVASVAEK) et/ou 61 à 85 (EQASHLGGAVFSGAGNIAAATGLVK) de la β-synucléine.

6. Procédé selon l'une quelconque des revendications précédentes, dans lequel la valeur seuil pour le diagnostic de la maladie d'Alzheimer est à 10 pg/ml de β-synucléine dans le sérum, de préférence à 10,6 pg/ml.

7. Procédé selon l'une quelconque des revendications précédentes, dans lequel la valeur seuil pour le diagnostic de la maladie de Creutzfeldt-Jakob est à 39,8 pg/ml, de préférence comme déterminé par Ser MRM (index de Youden).

8. Procédé selon l'une quelconque des revendications 1 à 4, dans lequel la détermination de la concentration en β-synucléine implique des réseaux mono-moléculaires (Simoa), de préférence la détermination de la concentration en β-synucléine est effectuée au moyen de réseaux mono-moléculaires, plus préférablement dans lequel une étape d'immunoprécipitation de β-synucléine est effectuée avant la détermination de la concentration en β-synucléine via des réseaux mono-moléculaires (Simoa).

9. Procédé selon la revendication 8, dans lequel la configuration des réseaux monomoléculaires utilise un anticorps monoclonal anti-β-synucléine couplé à des billes paramagnétiques carboxylées et/ou un anticorps monoclonal anti-α- et β-synucléine biotinylé comme anticorps de détection et/ou streptavidine-β-galactosidase (SBG) comme réactif enzymatique et/ou résorufine β-D galactopyranoside comme substrat enzymatique.

10. Procédé selon l'une quelconque des revendications précédentes, dans lequel le procédé est utilisé pour distinguer les unes des autres la maladie de Creutzfeldt-Jakob, la maladie d'Alzheimer, la démence frontotemporale à variante comportementale et la sclérose latérale amyotrophique, dans lequel le procédé combine la détermination de taux de neurofilaments (NfL) et de β-synucléine dans le sang, de préférence dans lequel les taux de neurofilaments (NfL) dans le sang sont augmentés par rapport à une valeur de référence pour des sujets sains.

11. Utilisation de la β-synucléine comme biomarqueur pour diagnostiquer ou évaluer le statut d'une maladie ou de maladies associées à la dégénérescence synaptique, de préférence pour évaluer la réponse à la thérapie d'un patient diagnostiqué de ou suspecté d'être atteint d'une maladie ou de maladies associées à la dégénérescence synaptique, et/ou pour classer un stade d'un patient diagnostiqué de ou suspecté d'être atteint d'une maladie ou de maladies associées à la dégénérescence synaptique, et/ou pour surveiller le contrôle d'une maladie chez un patient diagnostiqué de ou suspecté d'être atteint d'une maladie ou de maladies associées à la dégénérescence synaptique, et/ou pour surveiller la progression d'une maladie chez un patient diagnostiqué de ou suspecté d'être atteint d'une maladie ou de maladies associées à la dégénérescence synaptique,
dans laquelle la maladie ou les maladies associées à la dégénérescence synaptique sont une ou plusieurs parmi la maladie d'Alzheimer, la maladie de Creutzfeldt-Jakob, les lésions cérébrales traumatiques, un accident vasculaire cérébral et/ou un gliome,
dans laquelle l'utilisation comprend la détermination de la concentration de β-synucléine dans un échantillon du patient, dans laquelle l'échantillon du patient est du sang, du sérum ou du plasma, et dans laquelle l'utilisation est *ex vivo.*

12. Utilisation selon la revendication 11, dans laquelle la β-synucléine est utilisée comme biomarqueur dans le sérum d'un patient.
